# EUROPEAN PATENT APPLICATION

(11) **EP 3 351 173 A1**
(43) Date of publication of application: **25.07.2018**
(21) Application number: 17152811.0
(22) Date of filing: 24.01.2017
(51) Int. Cl.: A61B 5/107, A61B 5/00

(54) **AN OEDEMA DETERMINATION APPARATUS AND ASSOCIATED METHODS**

(71) Applicant: Nokia Technologies Oy, 02610 Espoo (FI)
(72) Inventor: Lindholm, Harri, 00320 Helsinki (FI); Kärkkäinen, Leo, 00100 Helsinki (FI); Rajala, Satu, 02680 Espoo (FI)
(74) Representative: Potter Clarkson LLP

(57) **Abstract**

An apparatus (100) based on at least a first limb strain measurement from a first strain sensor (103) and a second limb strain measurement from a second strain sensor (104), the first strain sensor and the second strain sensor forming part of one or more garments (101) for wearing on one or more of the limbs of a person, the first strain sensor and the second strain sensor configured to be positioned at different locations on the one or more limbs of the person; configured to provide for determination of a limb swelling indicator comprising a function of the first limb strain measurement and the second limb strain measurement, the limb swelling indicator thereby indicative of a distribution of swelling over the one or more of the limbs of the person.

## Description

### Technical Field

The present disclosure relates to the field of strain measurement of the limb(s) of a person for identification of oedema and associated methods, computer programs and apparatus. Certain disclosed aspects/examples relate to portable electronic devices, in particular, so-called hand-portable electronic devices which may be hand-held in use (although they may be placed in a cradle in use). Such hand-portable electronic devices include so-called Personal Digital Assistants (PDAs), mobile telephones, smartphones and other smart devices, smartwatches and tablet PCs.

The portable electronic devices/apparatus according to one or more disclosed aspects/embodiments may provide one or more audio/text/video communication functions (e.g. tele-communication, video-communication, and/or text transmission (Short Message Service (SMS)/Multimedia Message Service (MMS)/e-mailing) functions), interactive/non-interactive viewing functions (e.g. web-browsing, navigation, TV/program viewing functions), music recording/playing functions (e.g. MP3 or other format and/or (FM/AM) radio broadcast recording/playing), downloading/sending of data functions, image capture functions (e.g. using a (e.g. in-built) digital camera), and gaming functions.

### Background

Electronic devices are becoming more and more ubiquitous, with users interacting with electronics in many different ways. For example, people carry portable electronic devices with them in the form of smartphones, tablet computers, laptops, smart-watches and e-book readers. Electronic devices may be used to monitor health and to detect early signs of disorders.

Oedema, swelling caused by accumulation of fluid locally or in larger areas of the body, can be a sign and symptom of several clinically important disorders. In an ageing population and for patients with cardiac disease, leg swelling might indicate problems in cardiac function, insufficient medication or some concomitant organ dysfunction. Among the young and healthy of the population, environmental heat load or venous insufficiency due to varicoses might provoke leg swelling. Long-term sitting, for example on long haul flights, can cause leg thrombosis, which is usually reflected by unilateral leg swelling. Apparatus for detecting and measuring such leg oedema, as well as oedema in other limbs, may provide for various technical effects, as described herein.

The listing or discussion of a prior-published document or any background in this specification should not necessarily be taken as an acknowledgement that the document or background is part of the state of the art or is common general knowledge. One or more aspects/examples of the present disclosure may or may not address one or more of the background issues.

### Summary

In a first example aspect there is provided an apparatus comprising at least one processor and at least one memory including computer program code, the at least one memory and the computer program code configured to, with the at least one processor, cause the apparatus to perform at least the following: based on at least a first limb strain measurement from a first strain sensor and a second limb strain measurement from a second strain sensor, the first strain sensor and the second strain sensor forming part of one or more garments for wearing on one or more of the limbs of a person, the first strain sensor and the second strain sensor configured to be positioned at different locations on the one or more limbs of the person; provide for determination of a limb swelling indicator comprising a function of the first limb strain measurement and the second limb strain measurement, the limb swelling indicator thereby indicative of a distribution of swelling over the one or more of the limbs of the person.

In one or more embodiments, the limb swelling indicator may be based on the function comprising one or more of: a difference between the first limb strain measurement and the second limb strain measurement; a change in the first limb strain measurement relative to a change in the second limb strain measurement; a rate of change in the first limb strain measurement relative to a rate of change in the second limb strain measurement; a combination of the first limb strain measurement and the second limb strain measurement; a change in the first limb strain measurement in combination with a change in the second limb strain measurement; a rate of change in the first limb strain measurement in combination with a rate of change in the second limb strain measurement. In one or more examples, other functions may be used.

In one or more embodiments, the first limb strain measurement is from a first limb of a person and the second limb strain measurement is from a second limb, different to the first limb, of the person. A technical effect of this may be the determination of unilateral swelling, comprising different swelling across different limbs, or the identification of possible errors, such as due to posture, in the limb strain measurements if bilateral swelling is expected. In one or more embodiments, the first limb strain measurement and second limb strain measurement may be from, respectively, one of: (i) a left lower limb and a right lower limb; (ii) a left upper limb and a right upper limb; (iii) a left upper limb and a left lower limb; (iv) a right upper limb and a right lower limb; (v) a right upper limb and a left lower limb; (vi) a left upper limb and a right lower limb.

In one or more embodiments, the first limb strain measurement may be from a first location on a limb of a person and the second limb strain measurement is from a second location, spaced from the first location on the same limb of the person. The same limb may comprise an upper limb or lower limb. In such an example, the limb swelling indicator may be indicative of a contour or distribution of swelling over the limb or swelling at a plurality of locations that may be indicative of a medical condition, for example.

In one or more embodiments, the apparatus, based on the limb swelling indicator satisfying one or more predetermined alert conditions, may provide for one or more of generation of an alert to the person wearing the garment and the automatic sending of a message to a predetermined recipient.

Alert conditions may be indicative of acute changes, such as venous thrombosis, or worsening of a chronic disease (heart failure, kidney disease, hepatic failure, etc).

The generation of an alert may inform the wearer to take a predetermined specified action to relieve the oedema, to contact a medical professional, or to take another action. The predetermined recipient of the message may be a medical professional or an assistant of the wearer, for example, via a mobile communications device. In one or more examples, the apparatus comprises a mobile communications device and therefore the determination of the limb swelling indicator and the sending of the message may be performed by the mobile communication device.

The alert may comprise one or more of an audible alert, a visual alert and a haptic alert. An element for providing the alert based on signalling from the apparatus may be incorporated into the garment or the apparatus or be in communication with the apparatus. The element may comprise a speaker for an audible alert, an LED for a visual alert or a vibrator for a haptic alert.

The message may include one or more of: the limb swelling indicator; the first limb strain measurement; the second limb strain measurement; any combination of the limb swelling indicator, the first limb strain measurement and the second limb strain measurement; historical measurements of one or more of the limb swelling indicator, the first limb strain measurement and the second limb strain measurement over a predetermined time period. The predetermined time period may be a time up to 10 minutes, 30 minutes, 1 hour, 2 hours, 6 hours, 1 day or 1 week prior to limb swelling indicator satisfying the one or more predetermined alert conditions.

The alert condition may comprise the limb swelling indicator or an average thereof being above or below a predetermined threshold. The alert condition may comprise the limb swelling indicator being above or below a predetermined threshold for a predetermined time.

In a further aspect there is provided the apparatus in combination with the garment.

In one or more embodiments, the apparatus and the one or more garments having a configuration comprising one or more of: the one or more garments including the first and the second strain sensors and a wired connection to the apparatus; the one or more garments including the first and the second strain sensors associated with one or more transmitters and the apparatus being associated with a receiver for implementing a wireless connection between the one or more transmitters associated with the first and the second strain sensors and the receiver associated with the apparatus; the one or more garments including a hand operable release mechanism for releasably connecting one or more of the at least first and second strain sensors to the one or more garments; the one or more garments including the first and the second strain sensors associated with one or more transmitters for implementing a wireless connection between the first and second strain sensors and the apparatus and a hand operable release mechanism for releasably connecting the one or more transmitters to the one or more garments.

In one or more embodiments, one or more of the first and second strain sensors may comprise: a metal foil strain gauge; a semiconductor strain gauge; a nanoparticle based strain gauge; an elastomer strain gauge; a piezoelectric polymer strain gauge; a piezoelectric quartz strain gauge; a birefringent film strain gauge; a fiber-optic strain gauge; or a textile-based strain gauge.

In one or more embodiments, the garment may comprise one of: a sock having the first strain sensor and the second strain sensor at different locations therein; a pair of socks having the first strain sensor in a first sock of the pair of socks and the second strain sensor in a second sock of the pair of socks; a shoe having the first strain sensor and the second strain sensor at different locations therein; a pair of shoes having the first strain sensor in a first shoe of the pair of shoes and the second strain sensor in a second shoe of the pair of shoes; a stocking having the first strain sensor and the second strain sensor at different locations therein; a pair of stockings having the first strain sensor in a first stocking of the pair of stockings and the second strain sensor in a second stocking of the pair of stockings; a glove having the first strain sensor and the second strain sensor at different locations therein; a pair of gloves having the first strain sensor in a first glove of the pair of gloves and the second train sensor in a second glove of the pair of gloves; a sleeve having the first strain sensor and the second strain sensor at different locations therein; a pair of sleeves having the first strain sensor in a first sleeve of the pair of sleeves and the second strain sensor in a second sleeve of the pair of sleeves; a combination of two different garments selected from a sock, stocking, shoe, glove and sleeve wherein the first strain sensor is in a first garment of the two garments and the second strain sensor is in a second garment of the two garments.

In one or more embodiments, the limb swelling indicator may be based on a function including further limb strain measurements from respective further strain sensors of the one or more garments. The garment may include further strain sensors. The further strain sensors may be incorporated into any of the previously disclosed locations or types of garment.

In one or more embodiments, the first strain sensor and the second strain sensor may be configured to be located at two different locations on the same limb or at corresponding locations on different limbs selected from any combination of: a thigh; upper/lower thigh; a calf; upper/lower calf; a foot; a heel of the foot; a ball of the foot; a dorsal part of the foot; a sole of the foot; a hand; an upper/lower forearm; an upper/lower upper arm.

In one or more embodiments, the first strain sensor and the second strain sensor may be configured to be located at two different locations on different limbs.

The location of the swelling may indicate the stage of a pathological process. Among chronically ill patients, the progress of the dysfunction is individual and combining the information of previous events and present findings may provide for the technical effect of the acceleration of the findings of right corrective measures. The location and information of local temperatures may be used to evaluate the nature of the disorder.

In a second aspect there is provided a method, the method based on at least a first limb strain measurement from a first strain sensor and a second limb strain measurement from a second strain sensor, the first strain sensor and the second strain sensor forming part of one or more garments for wearing on one or more of the limbs of the person, the first strain sensor and the second strain sensor configured to be positioned at different locations on the one or more limbs of the person; providing for determination of a limb swelling indicator as a function of the first limb strain measurement and the second limb strain measurement, the limb swelling indicator thereby indicative of a distribution of swelling over one or more of the limbs of the person.

In a third aspect there is provided a computer readable medium comprising computer program code stored thereon, the computer readable medium and computer program code being configured to, when run on at least one processor, perform at least the following: based on at least a first limb strain measurement from a first strain sensor and a second limb strain measurement from a second strain sensor, the first strain sensor and the second strain sensor forming part of one or more garments for wearing on one or more of the limbs of the person, the first strain sensor and the second strain sensor configured to be positioned at different locations on the one or more limbs of the person;
providing for determination of a limb swelling indicator as a function of the first limb strain measurement and the second limb strain measurement, the limb swelling indicator thereby indicative of a distribution of swelling over one or more of the limbs of the person.

In a further aspect there is provided a garment configuration comprising one or more garments configured to be worn on one or more of the limbs of a person, the one or more garments comprising a first strain sensor for providing a first limb strain measurement and a second strain sensor for providing a second limb strain measurement, the first strain sensor and the second strain sensor forming part of the one or more garments, the first strain sensor and the second strain sensor configured to be positioned at different locations on the one or more limbs of the person when the one or more garments are worn by the person.

In one or more embodiments, the one or more garments include a first sensor array comprising a plurality of strain sensors including the first strain sensor and a second sensor array comprising a plurality of strain sensors including the second strain sensor, the first sensor array located in the one or more garments for positioning the sensors thereof at different locations across a lower leg of the limbs, wherein the limbs are lower limbs, of the person and the second sensor array located in the one or more garments for positioning the sensors thereof at different locations across a foot of the lower limbs of the person.

In a further aspect there is provided an apparatus, the apparatus, based on at least a first limb strain measurement from a first strain sensor and a second limb strain measurement from a second strain sensor, the first strain sensor and the second strain sensor forming part of one or more garments for wearing on one or more of the limbs of the person, the first strain sensor and the second strain sensor configured to be positioned at different locations on the one or more limbs of the person;
comprising means to provide for determination of a limb swelling indicator as a function of the first limb strain measurement and the second limb strain measurement, the limb swelling indicator thereby indicative of a distribution of swelling over one or more of the limbs of the person.

The present disclosure includes one or more corresponding aspects, examples or features in isolation or in various combinations whether or not specifically stated (including claimed) in that combination or in isolation. Corresponding means and corresponding functional units (e.g., function enabler, object creator, measurer, and indicator calculator) for performing one or more of the discussed functions are also within the present disclosure.

Corresponding computer programs for implementing one or more of the methods disclosed are also within the present disclosure and encompassed by one or more of the described examples.

The above summary is intended to be merely exemplary and non-limiting.

### Brief Description of the Figures

A description is now given, by way of example only, with reference to the accompanying drawings, in which:
figure 1 illustrates an example apparatus embodiment comprising a number of electronic components, including memory and a processor, an example garment and example first and second strain sensors, according to one embodiment of the present disclosure;
figure 2 illustrates examples of the locations on the lower limbs along which strain sensors may be distributed by virtue of their position in the one or more garments, according to another embodiment of the present disclosure;
figure 3 illustrates an example garment comprising a plurality of strain sensors forming at least one strain sensor array, according to another embodiment of the present disclosure;
figure 4 illustrates a flowchart according to a method of the present disclosure; and
figure 5 illustrates schematically a computer readable medium providing a program.

### Description of Example Aspects

A technical effect of taking automatic strain measurements may be the early detection of leg swelling, thereby allow for early treatment. Possible technical effects of taking multiple strain measurements at different locations over the limbs of a patient may be that the accuracy of swelling detection is improved, the detection of false indications is reduced, the need for a measuring tape is avoided or a contour of swelling along a limb portion may be identified. Alternatively, the comparison of multiple strain measurements may allow for more accurate diagnosis of the cause of swelling.

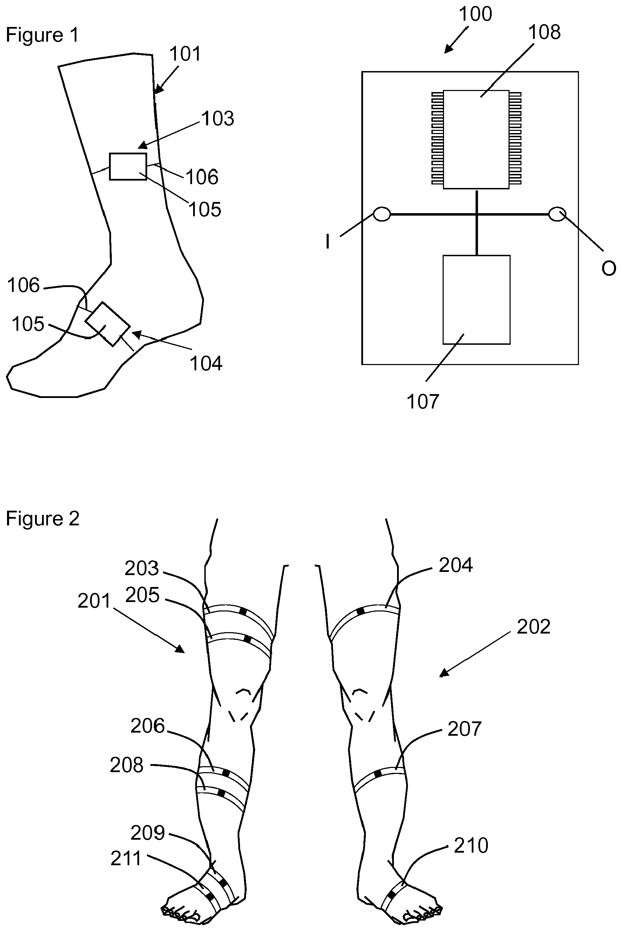

Figure 1 shows an apparatus 100 for determination of a limb swelling indicator based on a first limb strain measurement from a first strain sensor 103 and a second limb strain measurement from a second strain sensor 104, the first strain sensor and the second strain sensor forming part of one or more garments 101, which in this example comprises a sock, for wearing on one or both of the lower limbs of a person. By virtue of the location of the first strain sensor 103 and the second strain sensor 104 in the one or more garments 101, the sensors 103, 104 are configured to be positioned at different locations on the one or both lower limbs of the person.

Some embodiments may provide the technical effect of allowing for the determination of the limb swelling indicator to identify a distribution of swelling in one limb or across two or more limbs. Accordingly, the distribution of swelling over two legs and/or two arms and/or one arm and one leg may be determined. The limb may comprise a lower limb, e.g. a leg, and may include the foot of said lower limb. The limb may comprise an upper limb, e.g. an arm, and may or may not include the hand of said limb. Detecting the distribution of swelling may allow for the early recognition of a various problems in cardiac function, circulation or in relation to insufficient or incorrect medication. The determination of the limb swelling indicator as a function of at least two differently positioned limb strain measurements, which may comprise the measurement of the circumference of the limb at different longitudinal positions along the same limb or at corresponding/different longitudinal positions on different limbs, may provide for effective identification of problems.

The apparatus 100 comprises memory 107, a processor 108, input I and output O. In this embodiment only one processor and one memory are shown but it will be appreciated that other embodiments may utilise more than one processor and/or more than one memory (e.g. same or different processor/memory types).

In this embodiment the apparatus 100 is an Application Specific Integrated Circuit (ASIC) for a portable electronic device with a touch sensitive display. In other embodiments the apparatus 100 can be a module for such a device, or may be the device itself, wherein the processor 108 is a general purpose CPU of the device and the memory 107 is general purpose memory comprised by the device.

The input I allows for receipt of signalling to the apparatus 100 from further components, such as receipt of signalling to the apparatus 100 from first and/or second strain sensors 103, 104 incorporated in the one or more garments 101 via a wired or wireless connection. The output O allows for onward provision of signalling from within the apparatus 100 to further components such as a display screen, speaker, or vibration module. The output signalling may be configured to generate an alert or send a message. In this embodiment the input I and output O are part of a connection bus that allows for connection of the apparatus 100 to further components.

The processor 108 is a general purpose processor dedicated to executing/processing information received via the input I in accordance with instructions stored in the form of computer program code on the memory 107. The output signalling generated by such operations from the processor 108 is provided onwards to further components via the output O.

The memory 107 (not necessarily a single memory unit) is a computer readable medium (solid state memory in this example, but may be other types of memory such as a hard drive, ROM, RAM, Flash or the like) that stores computer program code. This computer program code stores instructions that are executable by the processor 108, when the program code is run on the processor 108. The internal connections between the memory 107 and the processor 108 can be understood to, in one or more example embodiments, provide an active coupling between the processor 108 and the memory 107 to allow the processor 108 to access the computer program code stored on the memory 107.

In this example the input I, output O, processor 108 and memory 107 are all electrically connected to one another internally to allow for electrical communication between the respective components I, O, 107, 108. In this example the components are all located proximate to one another so as to be formed together as an ASIC, in other words, so as to be integrated together as a single chip/circuit that can be installed into an electronic device. In other examples one or more or all of the components may be located separately from one another.

The first strain sensor 103 and the second strain sensor 104 are suitable for incorporation into a garment or a plurality of garments. The strain sensors 103, 104, may each comprise a sensor body 105 and a tape, strap or band 106 for extending circumferentially around parts of the limbs for measurement of swelling in a localized area of the limb where they are located. The strain sensor or tape, strap or band thereof may extend entirely around a limb or, alternatively, may extend only partially around the limb. The sensor body 105 may include a communication element to provide the measured limb strain measurement to the apparatus 100.

The one or more garments 101 including the first and second strain sensors 103, 104 may comprise a wired or wireless connection to the apparatus 100. The wired connection between the garment and the apparatus 100 may extend from one or more of the sensors 103, 104 to the input I of the apparatus 100.

In one or more embodiments, the garment 101 may include the first and second strain sensors 103, 104 and one or more transmitters (not shown) associated with the strain sensors. Thus, a transmitter may be provided for two or more of the sensors 103, 104 such that they share the transmitter. The apparatus 100 may be associated with a receiver (not shown) for implementing a wireless connection between the transmitter(s) of the first and second strain sensors 103, 104 of the garment 101 and the receiver of the apparatus 100.

In one or more examples, each strain sensor 103, 104 may be in communication with a corresponding transmitter or a transceiver device. The apparatus 100 may be in communication with a receiver or transceiver device. The communication between the sensors 103, 104 and the apparatus 100 may be one-way or two-way via the transmitter/receiver or transceiver/transceiver respectively.

In one or more embodiments, the one or more garments may include a hand operable release mechanism for releasably connecting one or more of the at least first and second strain sensors 103, 104 to the one or more garments. A technical effect of this embodiment may be that it allows for washing of the garment, as the sensors are removable.

In one or more embodiments, a transmitter associated with one or more of the strain sensors 103, 104 for implementing a wireless connection between the sensors 103, 104 and the apparatus 100 may include a hand operable release mechanism for releasably connecting the transmitter to the garment. Thus, the sensors and/or transmitters/transceivers may be removable.

Each strain sensor 103, 104 may be based on resistive or piezoresistive, piezoelectric, capacitive or optical sensing principle, such as metal foil, semiconductor (e.g. single crystal or polysilicon), nanoparticle based, elastomer, piezoelectric polymer (e.g. polyvinylidene fluoride, PVDF), piezoelectric quartz, birefringent film, fiber-optic strain gauges or textile based strain sensors. The strain sensor may detect a measure of the strain around a limb by measurement of the force on the band 106 due to at least a localized increase in the volume of the limb.

In one or more embodiments, the strain sensor comprises a textile based strain sensor or "fabric strain sensor", which may be formed of conductive yarns. A textile based strain sensor may detect a measure of the strain around a limb in a similar manner to other strain sensors, i.e., the resistance of the conductive yarn in the textile strain sensor is changed as a function of strain.

The first and second strain sensors 103, 104 may be the same type or different types. In some embodiments, a particular type of strain sensor may be associated with a particular garment or particular part of the limb, such as a foot, while a different type of strain sensor may be preferred for a different garment or for measuring a different part of a limb, such as the thigh, for example.

Returning to the apparatus 100, the apparatus 100 is, based on at least a first limb strain measurement from the first strain sensor 103 and a second limb strain measurement from the second strain sensor 104, configured to provide for determination of a limb swelling indicator comprising a function of the first limb strain measurement and the second limb strain measurement, the limb swelling indicator thereby indicative of a distribution of swelling over one or both of the lower limbs of the person. Equally, the limb swelling indicator may be indicative of a distribution of swelling over one or both of the upper limbs or the person or a combination of upper and lower limbs of the person.

It will be understood that the phrase lower limb may refer to any portion between the bottom of the hip extending downwards to the end of the foot. This may include one or more of, the thigh, the knee, the calf, the ankle or the foot. Of course, this may also include more specific portions of these body parts, such as, upper, mid or lower parts of the thigh and calf and in the case of a foot: a heel of the foot; a ball of the foot; a dorsal part of the foot; or the sole of the foot.

It will be further understood that the phrase upper limb may refer to any portion between the bottom of the shoulder extending downwards to the end of the hand. This may include one or more of the upper arm, the forearm, the wrist or the hand. Of course, this may also include more specific portions of these body parts, such as upper, mid or lower parts of the upper arm and the forearm and in the case of a hand: a heel of the hand or the palm of the hand.

Although swellings are often prominent in lower limbs, significant accumulation of fluid in the upper limbs is not uncommon in some special conditions, such as disturbed lymphatic circulation caused by breast cancer or its treatment.

The determination of the limb swelling indicator based on limb strain measurements from one or more of these portions of the limbs may be indicative of specific medical problems and, thus, may provide for the technical effect of providing an early warning of the onset of such a problem.

The limb swelling indicator may be based on one or more of a number of different functions of the first limb strain measurement and the second limb strain measurement. For example, the limb swelling indicator may be based on a difference between the first limb strain measurement and the second limb strain measurement. In this example, the comparison of the first limb strain measurement to the second limb strain measurement may allow for a measurement of the deviation of a first limb circumference to a second limb circumference. This may provide for accurate detection of swelling in one limb. Alternatively, if both sensors are located on the same limb, a difference in strain or the combined strain at two different locations may provide information relating to a localisation of swelling.

In another example, the limb swelling indicator may be based on a change in the first limb strain measurement relative to a change in the second limb strain measurement. In a situation where a similar change in strain in the locality of each sensor is anticipated, taking a measurement of the relative change in the strains measured by each strain sensor may allow for the detection of abnormal strain variation. An example of when this may occur could be when standing from a sitting position, the strain around both thighs may be expected to vary identically as the muscles work. Thus, if the relative change between the two limbs is beyond a predetermined threshold, this may actually be an indication of swelling occurring.

In another example, the limb swelling indicator may be based on a rate of change in the first limb strain measurement relative to a rate of change in the second limb strain measurement. In this case, a deviation in the relative rates of change of the limb strain measurements may be indicative of a sudden swelling in the vicinity of one of the two strain sensors.

In any of the above examples, the limb swelling indicator may also be a function of time. For instance, an instantaneous or short-lived change in one or more limb strain measurements of one or more sensor may be a result of the flexing of a muscle or movement, as opposed to swelling. Thus, the apparatus may be configured to average a plurality of first and second limb strain measurements over a predetermined period of time, such that the limb swelling indicator is an average function of the limb strain measurements over a period of time.

In some embodiments, based on the limb swelling indicator satisfying one or more predetermined alert conditions, the apparatus 100 may provide for one or more of generation of an alert to the wearer of the garment 101 and the automatic sending of a message to a predetermined recipient.

This may alert the wearer to take a specified action to relieve the oedema, to contact a medical professional, or to take another action. The alert may be sent directly to a medical professional or an assistant of the wearer, for example, via a mobile communications device.

The signalling at the output O may provide for generation of the visual, audible or haptic alert. A visual alert may comprise a flashing or constant light output. Alternatively, the visual alert may take the form of a message displayed on the screen of an electronic device which brings the user's attention to the reason for the alert.

A haptic alert may provide a vibration to alert the person. A device that provides the visual, audible or haptic alert may comprise a part of the garment 101 or it may alternatively comprise part of an electronic device of which the apparatus 100 also forms part. For example, a mobile phone may be caused to vibrate in response to the limb swelling indicator satisfying the alert conditions.

In another embodiment, the alert may be provided to a predetermined person, such as a medical practitioner in addition to or instead of to the person wearing the garment 101.

It will be understood that the apparatus 100 may take a range of different forms which are suitable for providing the determination of the limb swelling indicator. The apparatus 100 may be one or more of: a portable electronic device, a laptop computer, a mobile phone, a Smartphone, a tablet computer, a personal digital assistant, a digital camera, a smartwatch, smart eyewear, a pen based computer, a non-portable electronic device, a desktop computer, a monitor, a household appliance, a smart TV, a server, a wearable device or a module/circuitry for one or more of the same.

Figure 2 illustrates example positions at which the first and second strain sensors 103, 104 may be disposed along the length of a right lower limb 201 and a left lower limb 202. The first and second strain sensors form part of a garment, such as a sock, stocking, shoe or leggings, but for reasons of clarity no garments are shown in figure 2. The positioning of the first and second strain sensors in the garment provides for positioning on the lower limbs of the person when the garment is worn. Thus, the positions of the strain sensors in Figure 2 illustrate the positons at which the wearing of the associated garment places the sensor on the lower limbs 201, 202. It will be understood that in an alternative embodiment, the garment may be configured to be worn to extend over the upper limbs thereby providing for placement of the sensor(s) on the upper limbs.

In a first example, the first strain sensor comprises sensor 203 and the second strain sensor comprises sensor 204. Accordingly, the first limb strain measurement may be from a thigh of the right lower limb 201 of the person and the second limb strain measurement may be from a thigh of the left lower limb 202 of the person. In this example the strain sensors 203, 204 of figure 2 are disposed in corresponding positions on the two limbs.

In a second example, the first strain sensor comprises sensor 206 and the second strain sensor comprises sensor 207. Accordingly, the first limb strain measurement may be from a calf of the right lower limb 201 of the person and the second limb strain measurement may be from a calf of the left lower limb 202 of the person.

In a third example, the first strain sensor comprises sensor 209 and the second strain sensor comprises sensor 210. Accordingly, the first limb strain measurement may be from a foot of the right lower limb 201 of the person and the second limb strain measurement may be from a foot of the left lower limb 202 of the person.

Arrangement of pairs of sensors at corresponding positions on different limbs may allow for direct comparison between the limbs in order to identify undesirable unilateral swelling. Thus the limb swelling indicator may be indicative of the distribution of swelling across different limbs.

In other examples, the first and second strain sensors may be in different locations on different limbs. Thus, the first strain sensor may comprise sensor 203 and the second strain sensor may comprise sensor 207. Thus, the limb swelling indicator may be indicative of the distribution of swelling across the thigh of one leg and the calf of the other leg.

In another example, the first limb strain measurement may be from a first location on the limb of a person and the second limb strain measurement may be from a second location, spaced from the first location on the same limb of the person.

Examples of such an arrangement of first and second strain sensor pairs are also shown in figure 2. Example pairs may be, for example, first strain sensor 203 and the second strain sensor 205. Such limb strain measurements may provide a limb swelling indicator indicative of a swelling distribution across the thigh.

Measurements from first and second strain sensors comprising sensors 206 and 208 may provide for a limb swelling indicator indicative of a swelling distribution across the calf.

Measurements from first and second strain sensors comprising sensors 209 and 211 may provide for a limb swelling indicator indicative of a swelling distribution across the foot.

The limb swelling indicator, in this case, may be based on the detection of a variation in the swelling across two locations or a comparison of a change or rate of change of swelling between the locations of the first strain sensor and the second strain sensor (or an average thereof over time).

The first strain sensor and the second strain sensor, when disposed on the same limb may not need to be located in the same general region of the limb, such as in pair 203, 205, pair 206, 208 or pair 209, 211. In some embodiments, measurements from first and second strain sensors in different regions of the same limb may be used in order to determine a limb swelling indicator. For example, the first strain sensor may be sensor 203 on the thigh and the second strain sensor may be sensor 206 on the calf.

When taking actions such as walking, sitting or standing, for example, muscles work in known patterns in order to achieve the desired effect. By taking measurements from strain sensors distributed at different portions of the limb, predetermined variations in strain measurements may be known to correspond to particular actions of movement. Accordingly, the apparatus may be configured to use two or more of the limb strain measurements to determine the limb swelling indicator and one or more of the limb strain measurements to identify a non-swelling related user action (such as sitting, running, walking, cross-legged). In one or more examples, the apparatus may be configured, at least in part based on measurement(s) from one or more non-strain sensors, to identify a non-swelling related user action. For example, a non-strain sensor may comprise a barometer or an accelerometer. The predetermined alert condition may be configured to take account of the limb swelling indicator in combination with the determined non-swelling related user action.

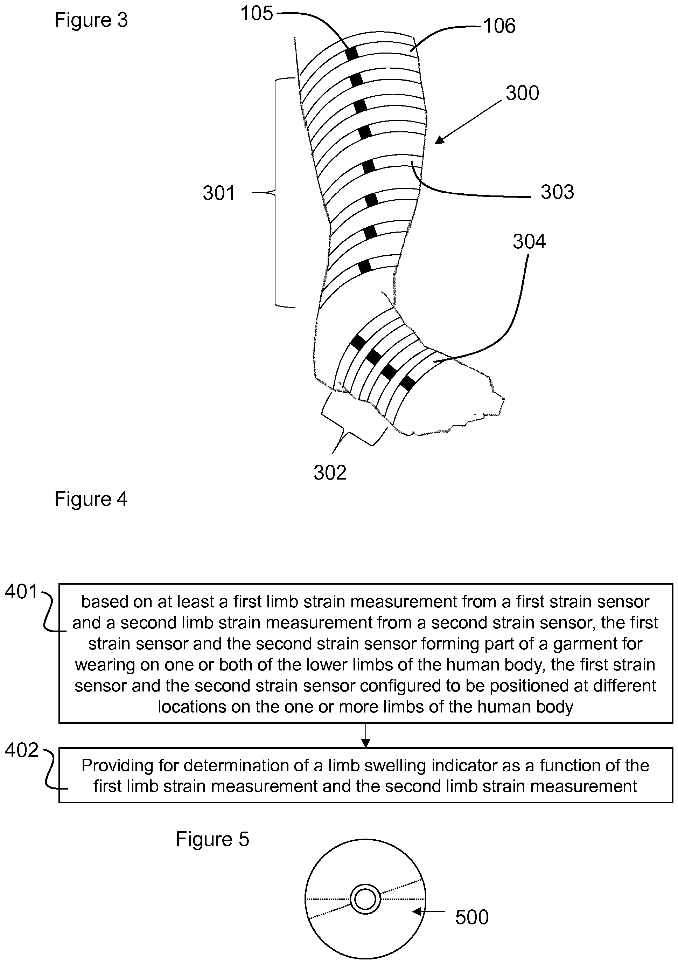

Figure 3 shows a garment 300 in the form of a calf-length sock comprising a plurality of sensors. As in figure 2, the sensors incorporated into this garment 300 comprise a fabric strap 106 and a sensor body 105. The sensors have been illustrated in an obvious manner in order to improve the clarity of the figure, however, it will be understood that the sensors may be more discretely incorporated into the fabric of the garment than shown in figure 3.

The one or more garments 300 are configured to be worn on one or more of the limbs of the person. As described above, the garment 300 comprises at least a first strain sensor for providing a first limb strain measurement and a second strain sensor for providing a second limb strain measurement.

In one or more examples, the garment 300 comprises a first sensor array 301 comprising a plurality of strain sensors each providing a respective limb strain measurement and including the first strain 303 sensor. For example, the sensor array 301 may comprise eight, or another number of strain sensors and may comprise a plurality of strain measurements so that the distribution of swelling over a particular section (e.g. thigh, calf, foot, etc) of the lower limb can be determined by way of a limb swelling indicator based on the measurements of the first sensor array 301.

While the garment 300 may include a single sensor 301 array in other examples, it may comprise two sensor arrays or more than two senor arrays. The sensor arrays may extend over different particular sections (e.g. thigh, calf, foot, upper arm, forearm, hand or upper, mid or lower portions thereof etc) of the limb.

Figure 3 shows the garment 300 comprising the first sensor array 301 and a second sensor array 302 comprising a plurality of strain sensors. The first sensor array 301 is located in the garment 300 for positioning of the sensors at different locations across the lower leg and the second sensor array 302 is located in the garment 300 for positioning the sensors thereof at different locations across the foot. Accordingly, the distribution of swelling across the leg and foot may be determined by respective limb swelling indicators. In other examples, the limb strain measurements of both the arrays 301, 302 may be combined by the function that determines the limb swelling indicator.

It will be understood that the arrays or sensors may be arranged in other locations along the limbs, such as the upper limbs or other locations on the lower limbs, but that Figure 3 provides an exemplary embodiment. A technical effect of this embodiment may be that the temporal and regional characterisation of swelling in one or more leg or between legs may allow for the assessment of the mechanisms and seriousness of changes. One or more embodiments may also be complemented by other physiological sensors, such as temperature sensors. In one or more examples, the limb swelling indicator may comprise a function that includes the temporal characteristics of the swelling at the different locations, such as the timing of when swelling (such above a threshold) develops at the different locations. In one or more examples, the limb swelling indicator may comprise a function that includes temperature or other physiological measures.

The one or more garments may comprise a number of different possible garments for covering at least a portion of one or more limbs.

For example, the garment 300 may comprise a pair of socks having the first strain sensor in a first sock of the pair of socks and the second strain sensor in a second sock of the pair of socks. Alternatively, a sock may have the first strain sensor 303 and the second strain sensor 304 at different locations therein. The socks may be: low-cut socks, which extend to below the ankle; ankle-dress socks, which extend to the lower or mid-calf; knee-length socks, which extend to just below the knee; or thigh socks, which extend generally to the mid-thigh.

In another example, the garment 300 may comprise a shoe having the first strain sensor 303 and the second strain sensor 304 at different locations therein. Alternatively, the garment 300 may comprise a pair of shoes having the first strain sensor 303 in a first shoe of the pair of shoes and the second strain sensor 304 in the second shoe of the pair of shoes. A shoe may comprise any one of a trainer; an ankle boot; a calf-high boot; a thigh-high boot; a heeled shoe or boot; a slipper; or any type of footwear.

In another example, the garment 300 may comprise a stocking having the first strain sensor 303 and the second strain sensor 304 at different locations therein. Alternatively, the garment 300 may comprise a pair of stockings having the first strain sensor 303 in a first stocking of the pair of stockings and the second strain sensor 304 in a second stocking of the pair of stockings. A stocking may be understood to mean a medical or compression stocking which may wrap any portion of the lower limb of a person, including but not limited to: the thigh, the knee, the calf, the ankle or the foot.

In another example, the garment 300 may comprise a glove for extending over at least a portion of the hand and, optionally, at least a portion of the arm. The glove may comprise the first the first strain sensor 303 and the second strain sensor 304 at different locations therein. Alternatively, the garment 300 may comprise a pair of gloves having the first strain sensor 303 in a first glove of the pair of gloves and the second strain sensor 304 in a second glove of the pair of gloves. A glove may be understood to mean a cold-weather glove, a fingered glove, a fingerless glove, a mitten, a sports gauntlet, driving gloves or another type of glove.

In yet another example, the garment 300 may comprise a sleeve having the first strain sensor 303 and the second strain sensor 304 at different locations therein. Alternatively, the garment 300 may comprise a pair of sleeves having the first strain sensor 303 in a first sleeve of the pair of sleeves and the second strain sensor 304 in a second sleeve of the pair of sleeves. A sleeve may be understood to be a garment which extends over any portion of the upper arm or forearm. For example, a short sleeve may extend from a T-shirt or short sleeved shirt and a long sleeve may extend from a shirt, a sweater or another garment. A sleeve may form a portion of a garment worn across the torso or it may be a separate garment entirely, such as a compression sleeve.

In yet another example, the garment 300 may comprise a combination of two different garments selected from a sock, stocking, shoe, glove and a sleeve wherein the first strain sensor 303 is in a first garment of the two garments and the second strain sensor 304 is in a second garment of the two garments.

Figure 4 shows a flow diagram illustrating the steps of: based on 401 at least a first limb strain measurement from a first strain sensor and a second limb strain measurement from a second strain sensor, the first strain sensor and the second strain sensor forming part of a garment for wearing on one or more of the limbs of the person, the first strain sensor and the second strain sensor configured to be positioned at different locations on the one or more limbs of the person; providing 402 for determination of a limb swelling indicator as a function of the first limb strain measurement and the second limb strain measurement, the limb swelling indicator thereby indicative of a distribution of swelling over one or more of the limbs of the person.

The step of providing 402 for determination of a limb swelling indicator as a function of the first limb strain measurement and the second limb strain measurement may comprise one or more of: determining a difference between the first limb strain measurement and the second limb strain measurement; determining a change in the first limb strain measurement relative to a change in the second limb strain measurement; determining a rate of change in the first limb strain measurement relative to a rate of change in the second limb strain measurement; determining a combination of the first limb strain measurement and the second limb strain measurement; determining a change in the first limb strain measurement in combination with a change in the second limb strain measurement; determining a rate of change in the first limb strain measurement in combination with a rate of change in the second limb strain measurement.

Figure 5 illustrates schematically a computer/processor readable medium 500 providing a program according to an example. In this example, the computer/processor readable medium is a disc such as a digital versatile disc (DVD) or a compact disc (CD). In other examples, the computer readable medium may be any medium that has been programmed in such a way as to carry out an inventive function. The computer program code may be distributed between the multiple memories of the same type, or multiple memories of a different type, such as ROM, RAM, flash, hard disk, solid state, etc.

Any mentioned apparatus and/or other features of particular mentioned apparatus may be provided by apparatus arranged such that they become configured to carry out the desired operations only when enabled, e.g. switched on, or the like. In such cases, they may not necessarily have the appropriate software loaded into the active memory in the non-enabled (e.g. switched off state) and only load the appropriate software in the enabled (e.g. on state). The apparatus may comprise hardware circuitry and/or firmware. The apparatus may comprise software loaded onto memory. Such software/computer programs may be recorded on the same memory/processor/functional units and/or on one or more memories/processors/ functional units.

In some examples, a particular mentioned apparatus may be pre-programmed with the appropriate software to carry out desired operations, and wherein the appropriate software can be enabled for use by a user downloading a "key", for example, to unlock/enable the software and its associated functionality. Advantages associated with such examples can include a reduced requirement to download data when further functionality is required for a device, and this can be useful in examples where a device is perceived to have sufficient capacity to store such pre-programmed software for functionality that may not be enabled by a user.

Any mentioned apparatus, circuitry, or processor 108 may have other functions in addition to the mentioned functions, and that these functions may be performed by the same apparatus, circuitry or processor 108. One or more disclosed aspects may encompass the electronic distribution of associated computer programs and computer programs (which may be source/transport encoded) recorded on an appropriate carrier (e.g. memory, signal).

Any "computer" described herein can comprise a collection of one or more individual processors/processing elements that may or may not be located on the same circuit board, or the same region/position of a circuit board or even the same device. In some examples one or more of any mentioned processors may be distributed over a plurality of devices. The same or different processor/processing elements may perform one or more functions described herein.

The term "signalling" may refer to one or more signals transmitted as a series of transmitted and/or received electrical/optical signals. The series of signals may comprise one, two, three, four or even more individual signal components or distinct signals to make up said signalling. Some or all of these individual signals may be transmitted/received by wireless or wired communication simultaneously, in sequence, and/or such that they temporally overlap one another.

With reference to any discussion of any mentioned computer and/or processor and memory (e.g. including ROM, CD-ROM etc), these may comprise a computer processor, Application Specific Integrated Circuit (ASIC), field-programmable gate array (FPGA), and/or other hardware components that have been programmed in such a way to carry out the inventive function.

The applicant hereby discloses in isolation each individual feature described herein and any combination of two or more such features, to the extent that such features or combinations are capable of being carried out based on the present specification as a whole, in the light of the common general knowledge of a person skilled in the art, irrespective of whether such features or combinations of features solve any problems disclosed herein, and without limitation to the scope of the claims. The applicant indicates that the disclosed aspects/examples may consist of any such individual feature or combination of features. In view of the foregoing description it will be evident to a person skilled in the art that various modifications may be made within the scope of the disclosure.

While there have been shown and described and pointed out fundamental novel features as applied to examples thereof, it will be understood that various omissions and substitutions and changes in the form and details of the devices and methods described may be made by those skilled in the art without departing from the scope of the disclosure. For example, it is expressly intended that all combinations of those elements and/or method steps which perform substantially the same function in substantially the same way to achieve the same results are within the scope of the disclosure. Moreover, it should be recognized that structures and/or elements and/or method steps shown and/or described in connection with any disclosed form or examples may be incorporated in any other disclosed or described or suggested form or example as a general matter of design choice. Furthermore, in the claims means-plus-function clauses are intended to cover the structures described herein as performing the recited function and not only structural equivalents, but also equivalent structures. Thus although a nail and a screw may not be structural equivalents in that a nail employs a cylindrical surface to secure wooden parts together, whereas a screw employs a helical surface, in the environment of fastening wooden parts, a nail and a screw may be equivalent structures.

## Claims

1. An apparatus comprising:
at least one processor; and
at least one memory including computer program code,
the at least one memory and the computer program code configured to, with the at least one processor, cause the apparatus to perform at least the following:
based on at least a first limb strain measurement from a first strain sensor and a second limb strain measurement from a second strain sensor, the first strain sensor and the second strain sensor forming part of one or more garments for wearing on one or more of the limbs of a person, the first strain sensor and the second strain sensor configured to be positioned at different locations on the one or more limbs of the person;
provide for determination of a limb swelling indicator comprising a function of the first limb strain measurement and the second limb strain measurement, the limb swelling indicator thereby indicative of a distribution of swelling over the one or more of the limbs of the person.

2. The apparatus of claim 1, wherein limb swelling indicator is based on the function comprising one or more of:
i) a difference between the first limb strain measurement and the second limb strain measurement;
ii) a change in the first limb strain measurement relative to a change in the second limb strain measurement;
iii) a rate of change in the first limb strain measurement relative to a rate of change in the second limb strain measurement;
iv) a combination of the first limb strain measurement and the second limb strain measurement;
v) a change in the first limb strain measurement in combination with a change in the second limb strain measurement;
vi) a rate of change in the first limb strain measurement in combination with a rate of change in the second limb strain measurement.

3. The apparatus of claim 1 or claim 2, wherein the first limb strain measurement is from a first limb of the person and the second limb strain measurement is from a second limb of the person, different to the first limb.

4. The apparatus of claim 1 or claim 2, wherein the first limb strain measurement is from a first location on the limb of the person and the second limb strain measurement is from a second location, spaced from the first location on the same limb of the person.

5. The apparatus of any preceding claim, wherein based on the limb swelling indicator satisfying one or more predetermined alert conditions, provide for one or more of generation of an alert to the person wearing the one or more garments and the automatic sending of a message to a predetermined recipient.

6. The apparatus of any preceding claim in combination with the one or more garments.

7. The combination of claim 6, wherein the apparatus and the one or more garments having a configuration comprising one or more of:
i) the one or more garments including the first and the second strain sensors and a wired connection to the apparatus;
ii) the one or more garments including the first and the second strain sensors associated with one or more transmitters and the apparatus being associated with a receiver for implementing a wireless connection from the one or more transmitters associated with the first and second strain sensors to the receiver associated with the apparatus;
iii) the one or more garments including a hand operable release mechanism for releasably connecting one or more of the at least first and second strain sensors to the one or more garments;
iv) the one or more garments including the first and the second strain sensors associated with one or more transmitters for implementing a wireless connection between the first and second strain sensors and the apparatus and a hand operable release mechanism for releasably connecting the one or more transmitters to the one or more garments.

8. The apparatus of any one of claims 1 to 5 or the combination of claim 6 or claim 7, wherein one or more of the first and the second strain sensors comprise:
i) a metal foil strain gauge;
ii) a semiconductor strain gauge;
iii) a nanoparticle based strain gauge;
iv) an elastomer strain gauge;
v) a piezoelectric polymer strain gauge;
vi) a piezoelectric quartz strain gauge;
vii) a birefringent film strain gauge;
viii) a fiber-optic strain gauge; or
ix) a textile-based strain gauge;

9. The apparatus of any one of claims 1 to 5 or the combination of any of claims 6 to 8, wherein the one or more garments comprises one of:
i) a sock having the first strain sensor and the second strain sensor at different locations therein;
ii) a pair of socks having the first strain sensor in a first sock of the pair of socks and the second strain sensor in a second sock of the pair of socks;
iii) a shoe having the first strain sensor and the second strain sensor at different locations therein;
iv) a pair of shoes having the first strain sensor in a first shoe of the pair of shoes and the second strain sensor in a second shoe of the pair of shoes;
v) a stocking having the first strain sensor and the second strain sensor at different locations therein;
vi) a pair of stockings having the first strain sensor in a first stocking of the pair of stockings and the second strain sensor in a second stocking of the pair of stockings;
vii) a glove having the first strain sensor and the second strain sensor at different locations therein;
viii) a pair of gloves having the first strain sensor in a first glove of the pair of gloves and the second strain sensor in a second glove of the pair of gloves;
ix) a sleeve having the first strain sensor and the second strain sensor at different locations therein;
x) a pair of sleeves having the first strain sensor in a first sleeve of the pair of sleeves and the second strain sensor in a second sleeve of the pair of sleeves;
xi) a combination of two different garments selected from a sock, stocking, shoe, glove and sleeve wherein the first strain sensor is in a first garment of the two garments and the second strain sensor is in a second garment of the two garments.

10. The apparatus of any one of claims 1 to 5 or the combination of any of claims 6 to 9, wherein the first strain sensor and the second strain sensor are configured to be located at two different locations on the same limb or at corresponding locations on different limbs selected from:
i) a thigh;
ii) a calf;
iii) a foot;
iv) a heel of the foot;
v) a ball of the foot;
vi) a dorsal part of the foot;
vii) a sole of the foot;
viii) a hand;
ix) a forearm;
x) an upper arm.

11. A garment configuration comprising one or more garments configured to be worn on one or more of the limbs of a person, the one or more garments comprising a first strain sensor for providing a first limb strain measurement and a second strain sensor for providing a second limb strain measurement, the first strain sensor and the second strain sensor forming part of the one or more garments, the first strain sensor and the second strain sensor configured to be positioned at different locations on the one or more limbs of the person when the one or more garments are worn by the person.

12. The garment configuration of claim 11, wherein the one or more garments include a first sensor array comprising a plurality of strain sensors including the first strain sensor and a second sensor array comprising a plurality of strain sensors including the second strain sensor, the first sensor array located in the one or more garments for positioning the sensors thereof at different locations across a lower leg of the limbs, wherein the limbs are lower limbs, of the person and the second sensor array located in the one or more garments for positioning the sensors thereof at different locations across a foot of the lower limbs of the person.

13. The apparatus of any one of claims 1 to 6, wherein the apparatus is one or more of: a portable electronic device, a laptop computer, a mobile phone, a Smartphone, a tablet computer, a personal digital assistant, a digital camera, a smartwatch, smart eyewear, a pen based computer, a non-portable electronic device, a desktop computer, a monitor, a household appliance, a smart TV, a server, a wearable device or a module/circuitry for one or more of the same.

14. A method, the method comprising
based on at least a first limb strain measurement from a first strain sensor and a second limb strain measurement from a second strain sensor, the first strain sensor and the second strain sensor forming part of one or more garments for wearing on one or more of the limbs of the person, the first strain sensor and the second strain sensor configured to be positioned at different locations on the one or more limbs of the person;
providing for determination of a limb swelling indicator as a function of the first limb strain measurement and the second limb strain measurement, the limb swelling indicator thereby indicative of a distribution of swelling over one or more of the limbs of the person.

15. A computer readable medium comprising computer program code stored thereon, the computer readable medium and computer program code being configured to, when run on at least one processor, perform at least the following:
based on at least a first limb strain measurement from a first strain sensor and a second limb strain measurement from a second strain sensor, the first strain sensor and the second strain sensor forming part of one or more garments for wearing on one or more of the limbs of the person, the first strain sensor and the second strain sensor configured to be positioned at different locations on the one or more limbs of the person;
providing for determination of a limb swelling indicator as a function of the first limb strain measurement and the second limb strain measurement, the limb swelling indicator thereby indicative of a distribution of swelling over one or more of the limbs of the person.
